# EUROPEAN PATENT APPLICATION

(11) **EP 0 989 192 A2**
(43) Date of publication of application: **29.03.2000**
(21) Application number: 99118116.5
(22) Date of filing: 10.09.1999
(51) Int. Cl.: C12Q 1/68

(54) **Method for synthesis of nucleic acids**

(30) Priority: 21.09.1998 JP 26679298; 21.09.1998 JP 26679398
(71) Applicant: Shimadzu Corporation, Kyoto-shi, Kyoto 604-8511 (JP)
(72) Inventor: Tonoike, Hiroshi, c/o Shimadzu Corporation, Kyoto-shi, Kyoto 604-8511 (JP); Kojima, Kouichi, c/o Shimadzu Corporation, Kyoto-shi, Kyoto 604-8511 (JP)
(74) Representative: WILHELMS, KILIAN & PARTNER Patentanwälte

(57) **Abstract**

The object of the present invention is to provide a novel method of efficiently amplifying DNA in a sample by suppressing the action of PCR inhibitory substances.

In the present method for synthesis of a desired gene in a sample, a substance for suppressing the action of PCR inhibitory substances is added to a reaction solution for gene amplification. For example, the action of PCR inhibitory substances is suppressed by the presence of sulfated polysaccharide, and/or dithiothreitol (DTT), in the reaction solution for gene amplification.

SEQUENCE LISTING
<110> shimadzu corp.
<120> Method for synthesis of nucleic acids
<130> G098929
<160> 4
<210> 1
<211> 24
<212> DNA

<213> Artificial Sequence

<400> 1

GAGCGGATAACAATTTCACACAGG

<210> 2

<211> 24

<212> DNA

<213> Artificial Sequence

<400> 2

CGCCAGGGTTTTCCCAGTCACGAC

<210> 3

<211> 20

<212> DNA

<213> Artificial Sequence

<400> 3

GAAGAGCCAAGGACAGGTAC

<210> 4

<211> 21

<212> DNA

<213> Artificial Sequence

<400> 4

GGAAAATAGACCAATAGGCAG

SEQUENCE LISTING
<110> shimadzu corp.
<120> Method for synthesis of nucleic acids
<130> G098929
<160> 4
<210> 1
<211> 24
<212> DNA
<213> Artificial Sequence
<400> 1
GAGCGGATAACAATTTCACACAGG

<210> 2
<211> 24
<212> DNA
<213> Artificial Sequence
<400> 2
CGCCAGGGTTTTCCCAGTCACGAC

<210> 3
<211> 20
<212> DNA
<213> Artificial Sequence
<400> 3
GAAGAGCCAAGGACAGGTAC

<210> 4
<211> 21
<212> DNA
<213> Artificial Sequence
<400> 4
GGAAAATAGACCAATAGGCAG

## Description

### BACKGROUND OF THE INVENTION

### Filed of the Invention

The present invention relates to a method for synthesis of nucleic acids and in particular to a method for synthesis of nucleic acids by polymerase chain reaction (abbreviated hereinafter to PCR).

### Disclosure of the Related Art

The PCR techniques are those capable of amplifying a target DNA fragment hundred thousand times by repeating the steps of dissociating a DNA chain into a single-stranded chain, hybridizing primers with regions containing a specific region in the DNA chain, and synthesizing DNA by DNA polymerase. The PCR techniques are described in Japanese Laid-open Patent Publication No.61-274697 that discloses the invention of Morris et al.

The PCR techniques can be utilized as a highly sensitive analysis method for nucleic acids in various samples and particularly utilized for diagnosis of nucleic acids in a sample derived from animal body fluid. Accordingly, the PCR techniques are utilized for diagnosis of infections, hereditary diseases, and cancers. Further, the PCR techniques are also suitable for examination of DNA typing in transplantation or in a paternity test. In these cases, peripheral blood is often selected as the object of examination.

One disadvantage of the PCR techniques is that the reaction is inhibited by pigments, proteins, sugars or unknown impurities. That is, it is well-known that many kinds of DNA polymerase including Thermus aquaticus-derived Taq DNA polymerase as a typical thermostable DNA polymerase are strongly inhibited if even trace impurities derived from body fluid are present in the PCR reaction solution.

Accordingly, DNA amplification by the PCR techniques should be preceded by the process of separating cells, bacteria, viruses etc. (referred to hereinafter as gene inclusion body) from a sample and then extracting nucleic acids from the gene inclusion body. For this process, the gene inclusion body is decomposed with an enzyme, a surface active agent, a chaotropic agent etc., and nucleic acids are extracted by e.g. phenol or phenol/chloroform from the decomposed gene inclusion body, as is carried in the prior art.

Recently, an ion-exchange resin, a glass filter, glass beads or a protein-aggregating reagent is used in the process of extracting nucleic acids.

However, even if nucleic acids in the sample are purified by these methods, there are many cases where complete removal of impurities is difficult while the amount of nucleic acids recovered in the sample is varied, and accordingly the subsequent synthesis of nucleic acid may fail particularly where the content of target nucleic acid in the sample is low. Further, the operation for these purification methods are complicated and time-consuming, and it is highly possible that contamination occurs during the procedure.

Accordingly, a more simple and effective sample pretreatment method has been desired to solve these problems.

### SUMMARY OF THE INVENTION

Accordingly, the object of the present invention is to provide a novel method in which DNA in a sample is amplified efficiently regardless of the type of sample by suppressing the action of PCR inhibitory substances.

Heparin used conventionally as a blood anticoagulant is known as PCR inhibitor and regarded as an undesirable substance to be added to a sample. However, as a result of eager study of these phenomena, we found that heparin suppresses the action of PCR inhibitory substances in a biological sample. Further, we found that not only heparin but also sulfated polysaccharides such as dextran sulfate exhibit similar action, thus arriving at the present invention.

To solve the problem described above, the present invention provides a method for synthesis of nucleic acids in a sample which comprises adding a substance for suppressing the action of PCR inhibitory substances to a reaction solution for gene amplification to amplify a desired gene in a sample.

In the method for synthesis of nucleic acids according to the present invention, sulfated polysaccharide and/or salts thereof (referred to hereinafter as sulfated polysaccharide collectively) are added to a reaction solution for gene amplification.

Here, the sulfated polysaccharide may be added first to a sample and then to a reaction solution for gene amplification, or may be added directly to a reaction solution for gene amplification. Further, the sulfated polysaccharide has the same effect even if it is contained heterogeneously in a reaction solution for gene amplification (for example in the case where sulfated polysaccharide is added to a sample and this sample is added without stirring to the reaction solution). Further, one kind or several kinds of sulfated polysaccharides may be used.

Preferable examples of sulfated polysaccharides include, but are not limited to, heparin and salts thereof and dextran sulfate and salts thereof, but it is further possible to use heparin sulfate, chondroitin sulfate, dermatan sulfate, funoran (phonetic transcription), sulfated agarose, carragheenan, porphyran, fucoidan, sulfated curdlan etc.

Alternatively, dithiothreitol is added to a reaction solution for gene amplification in the method for synthesis of nucleic acids according to the present invention. Here, dithiothreitol (DTT) may be added first to a sample and then to a reaction solution for gene amplification, or may be added directly to a reaction solution for gene amplification. Further, DTT has the same effect even if it is contained heterogeneously in a reaction solution for gene amplification (for example in the case where DTT is added to a sample and this sample is added without stirring to the reaction solution).

DTT is added in a concentration of 0.1 mM or more, preferably 0.25 to 2 mM to the reaction solution for gene amplification.

In the present invention, both sulfated polysaccharide and DTT may be added to a reaction solution for gene amplification.

According to the present invention, it becomes possible to amplify desired DNA directly and efficiently from a sample containing a large amount of PCR inhibitory substances such as in serum, plasma and blood without requiring the step of separating and purifying nucleic acids. According to the present invention, it becomes possible to conduct the procedure of synthesizing nucleic acids easily and rapidly and to reduce the occurrence of contamination.

### BRIEF DESCRIPTION OF THE DRAWINGS

Fig. 1 is a profile in electrophoresis of a reaction solution after PCR where 1 µl human plasma treated with citric acid, and heparin at a varying concentration, were added to a PCR reaction solution (50 µl).
Fig. 2 is a profile in electrophoresis of a reaction solution after PCR where 0.8 µg/ml heparin achieving highly efficient amplification in Example 1 was added or not added to a reaction solution with serially diluted blood.
Fig. 3 is a profile in electrophoresis of a reaction solution after PCR where 1 µl human serum and dextran sulfate at a varying concentration were added to a PCR reaction solution (50 µl).
Fig. 4 is a profile in electrophoresis of a reaction solution after PCR where 0.05 µl human blood and dextran sulfate at a varying concentration were added to a PCR reaction solution (50 µl).

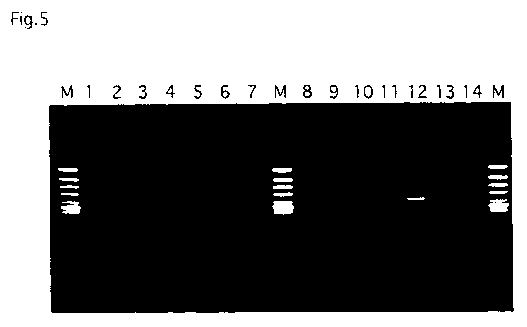

Fig. 5 is a profile in electrophoresis of a reaction solution after PCR where 2 or 1 µl human blood treated with citric acid, and DTT at a varying concentration, were added to a PCR reaction solution (50 µl).

### DETAILED DESCRIPTION OF THE INVENTION

The sample in the present invention is a gene inclusion body in a living body-derived sample or a sample itself derived from a living body, and the sample derived from a living body refers to animal and plant tissues, body fluid, excretions etc., and the gene inclusion body refers to cells, bacteria, viruses etc. The body fluid includes blood, saliva, cerebrospinal fluid, urine and milk, and the cells include, but are not limited to, leukocytes separated from blood. The gene inclusion body in a sample derived from a living body, or the sample derived from a living body, is added directly to a reaction solution for gene amplification without any special pretreatment.

The reaction solution for gene amplification usually contains a pH buffer solution, salts such as MgCl₂ and KCl, primers, deoxyribonucleotides and a thermostable enzyme. The salts used are replaced as necessary by other salts. Further, various materials including proteins such as gelatin and albumin, dimethyl sulfoxide, and surfactants are added in some cases.

The pH buffer solution is a combination of tris(hydroxymethyl)aminomethane and a mineral acid such as hydrochloric acid, nitric acid or sulfuric acid, among which hydrochloric acid is preferable. It is also possible to use various pH buffer solutions prepared by combining Tricine, CAPSO (3-N-cyclohexylamino-2-hydroxypropanesulfonic acid) or CHES (2-(cyclohexylamino) ethanesulfonic acid) with caustic soda or caustic potash. The pH-adjusted buffer solutions can be used at a concentration of 10 mM to 100 mM in a reaction solution for gene amplification.

The primer is an oligonucleotide serving as a start site of synthesis in the presence of nucleic acids, amplification reagents etc. The primer is preferably single-stranded, but a double-stranded primer can also be used. In the case of a double-stranded primer, the primer is preferably rendered single-stranded prior to the amplification reaction. The primer can be synthesized in any methods known in the art or can be isolated from the biological world.

The thermostable enzyme refers to an enzyme synthesizing nucleic acid by primer addition or to such chemical synthesis system. Suitable thermostable enzymes include, but are not limited to, E. coli DNA polymerase I, a Klenow fragment from E. coli DNA polymerase, T4 DNA polymerase, Taq DNA polymerase, T. litoralis DNA polymerase, Tth DNA polymerase, Pfu DNA polymerase, and reverse transcriptase.

Further, synergistic effect is achieved by adjusting the pH of the reaction solution for gene amplification. For example, its pH value is 8.1 or more, preferably 8.5 to 9.5 under the temperature condition of 25°C.

In the present invention, polyamine may also be added to the reaction solution for gene amplification.

The procedure for synthesis of nucleic acids according to the present invention is the same as in the conventional method except that a substance for suppressing the action of PCR inhibitors i.e. such as sulfated polysaccharide or DTT is added. That is, a target double-stranded DNA fragment to be amplified is converted into single-stranded DNA by thermal denaturation (denaturation step). Then, primers is hybridized with a region containing the fragment to be amplified (annealing step). Then, primer extension reaction is conducted by the action of DNA polymerase in the presence of 4 kinds of deoxyribonucleotides (dATP, dGTP, dCTP and dTTP) (polymerization step).

### EXAMPLES

### (Experimental Example 1)

After 1 µl of human plasma treated with citric acid was added to a PCR reaction solution (50 µl), PCR was conducted. 3 fg of PUC18 plasmid DNA was added to a PCR reaction solution (50 µl) as template DNA. The PCR primers used are an oligonucleotide (RV-M, SEQ ID NO:1) having a plus chain of PUC18 plasmid DNA and an oligonucleotide (M13-47, SEQ ID NO:2) having a minus chain of PUC18 plasmid DNA, and their sequences are as follows. As a result of PCR using these 2 primers, a 150 bp amplification product can be obtained.
RV-M: 5' GAGCGGATAACAATTTCACACAGG 3'
M13-47: 5' CGCCAGGGTTTTCCCAGTCACGAC 3'

The PCR reaction solution used was a solution consisting of 10 mM Tris-HCl, 50 mM KCl, 1.5 mM MgCl₂, 200 µM each of dATP, dCTP, dGTP and dTTP and 0.4 µM each of the primers, 1.25 units/50 µl Taq DNA polymerase (TaKaRa Taq, Takara Shuzo, Kyoto, Japan).

Polymerization involved preheating at 94°C for 3 minutes, 40 cycles each consisting of PCR reaction under the conditions of 94°C for 30 seconds, 55°C for 1 minute and 72°C for 1 minute, and final reaction at 72°C for 7 minutes. After PCR, 5 µl of the reaction solution was subjected to electrophoresis on 2.5% agarose gel in TAE (40 mM Tris-acetate, 1 mM EDTA) containing 0.5 µg/ml ethidium bromide to detect the amplification product.

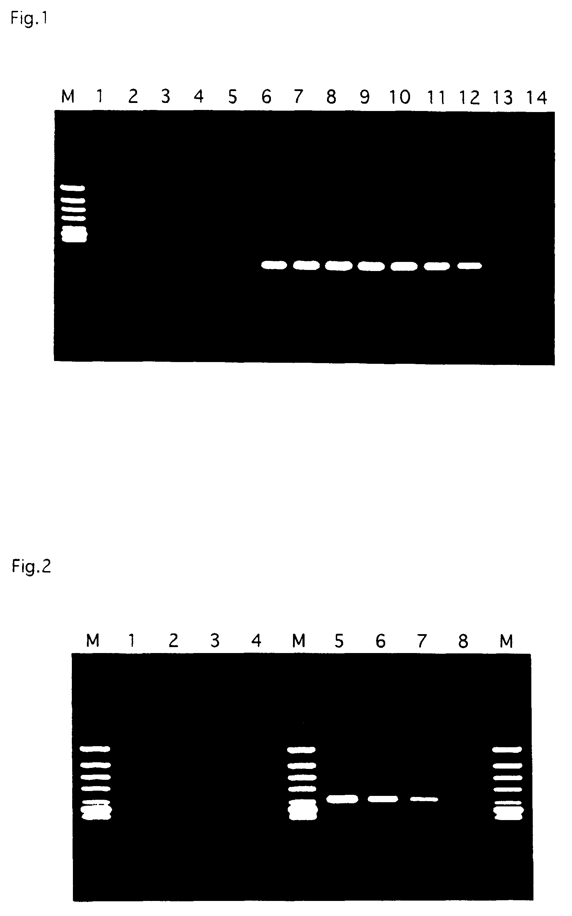

A profile in electrophoresis of the amplification product after PCR where sodium heparin (Wako Pure Chemical Industries) was added directly to the PCR reaction solution is shown in Fig. 1. In Fig. 1, M is a size marker (250 ng φX174-RF DNA cleaved with HincII); 1 is a sample with no heparin added; 2 is a sample with 0.025 µg/ml heparin added; 3, 0.05 µg/ml heparin added; 4, 0.1 µg/ml heparin added; 5, 0.2 µg/ml heparin added; 6, 0.4 µg/ml heparin added; 7, 0.8 µg/ml heparin added; 8, 1.6 µg/ml heparin added; 9, 3.2 µg/ml heparin added; 10, 6.4 µg/ml heparin added; 11, 12.8 µg/ml heparin added; 12, 25.6 µg/ml heparin added; 13, 51.2 µg/ml heparin added; and 14, 102.4 µg/ml heparin added.

As a result, it was found that the PCR amplification product could be obtained by adding heparin. It is preferable that heparin is added in an amount of 0.1 µg/ml or more, particularly 0.4 µg to 25.6 µg/ml.

### (Experimental Example 2)

In this example, PCR was conducted where 0.8 µg/ml heparin indicating highly efficient amplification in Example 1 was added or not added to a reaction solution with serially diluted blood. The composition of the PCR reaction solution, the conditions for PCR, and the conditions for electrophoresis after PCR were the same as in Experimental Example 1.

The PCR primers used are oligonucleotides (GH20, SEQ ID NO:3; and GH21, SEQ ID NO:4) which have plus and minus chains respectively located in a human β-globin gene, and their sequences are as follows. As a result of PCR using these 2 primers, a 408 bp amplification product can be obtained.
GH20: 5' GAAGAGCCAAGGACAGGTAC 3'
GH21: 5' GGAAAATAGACCAATAGGCAG 3'

The experiment result (electrophoretic profile) is shown in Fig. 2. In Fig. 2, M is a molecular weight marker; 1 & 5 are samples with 0.5% blood added; 2 & 6, 0.25% blood added; 3 & 7, 0.125% blood added; and 4 & 8, no blood added. 1 through 4 are samples with no heparin added, and 5 through 8 are samples with heparin added.

From Fig. 2, it can be seen that even if blood is used directly as the template, the action of PCR inhibitory substances can be suppressed by adding heparin and the amplification product can be detected highly sensitively.

### (Experimental Example 3)

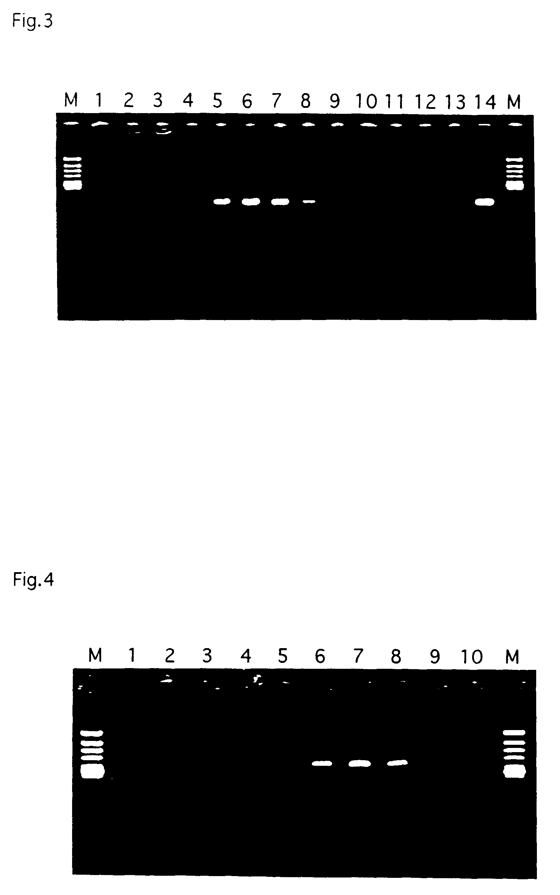

In this example, PCR was conducted after 1 µl of human serum was added to a PCR reaction solution (50 µl). 3 fg of PUC18 plasmid DNA was added to a PCR reaction solution (50 µl) as template DNA. As PCR primers, RV-M and M13-47 in Experimental Example 1 were also used. Dextran sulfate (average molecular weight, 5,500) in an amount ranging from 0.0023 ng to 100 µg was added, and the effect of dextran sulfate on recovery of the reaction by preventing human serum from inhibiting the PCR reaction. The composition of the PCR reaction solution used in the reaction and the conditions for electrophoresis after PCR were the same as in Experimental Example 1. Polymerization involved preheating at 94°C for 4.5 minutes, 40 cycles each consisting of PCR reaction under the conditions of 94°C for 30 seconds, 58°C for 1 minute and 72°C for 1 minute, and final reaction at 72°C for 7 minutes. The experiment results (electrophoretic profile) are shown in Fig. 3.

As a result, PUC18 plasmid DNA acts as the template to give a 150 bp reaction product in the case of 14 where neither human serum nor dextran sulfate are added. In the case of 13 where 1 µl of human serum is added to 50 µl of this reaction solution, the reaction is inhibited and the product is not detected. Further, even if dextran sulfate is added in amounts of 0.023 ng in the case of 12, 0.095 ng in the case of 11, 0.38 ng in the case of 10, and 1.52 ng in the case of 9, the reaction product is not detected, but if dextran sulfate is added in amounts of 6.1 ng in the case of 8, 24 ng in the case of 7, 97 ng in the case of 6, and 390 ng in the case of 5, the reaction product is detected due to the effect of dextran sulfate. Further, if dextran sulfate is added in amounts of 1.6 µg in the case of 4, 6.3 µg in the case of 3, 25 µg in the case of 2, and 100 µg in the case of 1, the product is not detected.

From the foregoing results, it was revealed that dextran sulfate suppresses the inhibitory action of serum on the reaction, thus recovering the reaction.

The concentrations at which the effect was observed in this example were in the range of 0.12 µg/ml to 7.8 µg/ml, but it was confirmed in other experiments that the effective concentration range is varied depending on the molecular weight of dextran sulfate used and the amount of serum added, so the effective concentration range is not limited to the range mentioned above.

### (Experimental Example 4)

In PCR reaction for amplifying human DNA contained in blood by adding 0.05 µl human blood and dextran sulfate at a varying concentration to 50 µl PCR reaction solution, the effect of raising reaction efficiency by the dextran sulfate was examined.

As PCR primers, GH20 and GH21 used in Experimental Example 2 were also used. The composition of the PCR reaction solution used in the reaction and the conditions for electrophoresis after PCR were the same as in Experimental Example 1.

0.05 µl of human blood and dextran sulfate (average molecular weight, 10,000) in a varying amount from 0.61 ng to 10 µg were added to this reaction solution. Polymerization involved preheating at 94°C for 4.5 minutes, 40 cycles each consisting of PCR reaction under the conditions of 94°C for 30 seconds, 55°C for 1 minute and 72°C for 1 minute, and final reaction at 72°C for 7 minutes. The experimental results (electrophoretic profile) are shown in Fig. 4.

As a result, no reaction product is obtained in the case of 2 where neither 0.05 µl blood nor dextran sulfate are added or in the case of 10 where 0.61 ng dextran sulfate is added, but a 408 bp reaction product is obtained by using human DNA in blood as the template in the presence of 2.44 ng dextran sulfate in the case of 9, 9.76 ng in the case of 8, 39 ng in the case of 7, and 156 ng in the case of 6. Further, the product is not detected in the presence of 0.625 µg dextran sulfate in the case of 5, 2.5 µg in the case of 4, and 10 µg in the case of 3. In the case of 1, blood is not added, so the reaction product can naturally not be obtained.

From the foregoing results, it was revealed that dextran sulfate has the effect of increasing reaction efficiency in PCR using blood as a sample.

The concentrations at which the effect was observed in this example were in the range of 0.049 µg/ml to 3.1 µg/ml, but it was confirmed from other experiments that the effective concentration range is varied depending on the molecular weight of dextran sulfate used and the amount of blood added, so the effective concentration range is not limited to the range mentioned above.

### (Experimental Example 5)

After 2 or 1 µl of human blood treated with citric acid was added to a PCR reaction solution (50 µl), PCR was conducted. The PCR primers used are oligonucleotides (GH20 & GH21) which have plus and minus chains respectively located in a human β -globin gene, and their sequences are as follows. As a result of PCR using these 2 primers, a 408 bp amplification product can be obtained.
GH20: 5' GAAGAGCCAAGGACAGGTAC 3'
GH21: 5' GGAAAATAGACCAATAGGCAG 3'

The reaction solution used is a solution consisting of 10 mM Tris-HCl, pH 8.3, 50 mM KCl, 1.5 mM MgCl₂, 200 µM each of dATP, dCTP, dGTP and dTTP and 0.4 µM each of the primers, 1.25 units/50 µl Taq DNA polymerase (TaKaRa Taq: Takara Shuzo, Kyoto, Japan).

Polymerization involved preheating at 94°C for 3 minutes, 40 cycles each consisting of PCR reaction under the conditions of 94°C for 30 seconds, 55°C for 1 minute and 72°C for 1 minute, and final reaction at 72°C for 7 minutes. After PCR, 5 µl of the reaction solution was subjected to electrophoresis on 2.5% agarose gel in TAE (40 mM Tris-acetate, 1 mM EDTA, pH 8.0) containing 0.5 µg/ml ethidium bromide to detect the amplification product.

A profile in electrophoresis of the amplification product after PCR where DTT was added directly to the PCR reaction solution is shown in Fig. 1. In Fig. 1, M is a size marker (250 ng φX174-RF DNA cleaved with HincII); 1 & 8 are samples with 0% DTT; 2 & 9, samples with 0.125 mM DTT added; 3 & 10, 0.25 mM DTT added; 4 & 11, 0.5mM DTT added; 5 & 12, 1 mM DTT added; 6 & 13, 2 mM DTT added; and 7 & 14, 4 mM DTT added. Further, 1 through 7 are samples with 2 µl blood added, and 8 through 14 are samples with 1 µl blood added.

As a result, it was found that the PCR amplification product is obtained by adding DTT. It is particularly preferable to add 1 mM DTT.

## Claims

1. A method for synthesis of nucleic acids, which comprises adding a substance for suppressing the action of PCR inhibitors to a reaction solution for gene amplification to amplify a desired gene in a sample.

2. A method for synthesis of nucleic acids according to claim 1, wherein sulfated polysaccharide and/or salts thereof (referred to hereinafter as sulfated polysaccharide collectively) to the reaction solution for gene amplification.

3. A method for synthesis of nucleic acids according to claim 2, wherein the sulfated polysaccharide is selected from heparin and salts thereof and dextran sulfate and salt thereof.

4. A method for synthesis of nucleic acids according to claim 2, wherein the sample is a gene inclusion body in a living body-derived sample, or a living body-derived sample itself.

5. A method for synthesis of nucleic acids according to any one of claims 2 to 4, wherein the gene inclusion body in a living body-derived sample, or the living body-derived sample itself, is added directly as the sample to the reaction solution for gene amplification.

6. A method for synthesis of nucleic acids according to claim 1, wherein dithiothreitol (DTT) is added to the reaction solution for gene amplification.

7. A method for synthesis of nucleic acids according to claim 6, wherein dithiothreitol is added in a concentration of 0.1 mM or more to the reaction solution for gene amplification.

8. A method for synthesis of nucleic acids according to claim 6, wherein the sample is a gene inclusion body in a living body-derived sample, or a living body-derived sample itself.

9. A method for synthesis of nucleic acids according to any one of claims 6 to 8, wherein the gene inclusion body in a living body-derived sample, or the living body-derived sample itself, is added directly as the sample to the reaction solution for gene amplification.
